# FASCICULE DE BREVET EUROPEEN

(11) **EP 3 481 812 B1**
(45) Date de publication et mention de la délivrance du brevet: **09.03.2022**
(21) Numéro de dépôt: 17742499.1
(22) Date de dépôt: 06.07.2017
(51) Int. Cl.: C07D 307/46, A61K 31/365, A61K 8/49

(54) **PROCÉDÉ DE TRANSFORMATION DE LA LÉVOGLUCOSÉNONE EN 4-HYDROXYMÉTHYLBUTYROLACTONE ET 4-HYDROXYMÉTHYLBUTÉNOLIDE EN ABSENCE DE SOLVANT ORGANIQUE ET DE CATALYSEUR**
VERFAHREN ZUR UMWANDLUNG VON LEVOGLUCOSENON IN 4-HYDROXYMETHYL-BUTYROLACTON UND 4-HYDROXYMETHYL-BUTENOLID OHNE VERWENDUNG EINES BELIEBIGEN ORGANISCHEN LÖSUNGSMITTELS UND KATALYSATORS
METHOD FOR CONVERTING LEVOGLUCOSENONE INTO 4-HYDROXYMETHYL BUTYROLACTONE AND 4-HYDROXYMETHYL BUTENOLIDE WITHOUT USING ANY ORGANIC SOLVENT AND CATALYST

(30) Priorité: 07.07.2016 FR 1656565
(43) Date de publication de la demande: 15.05.2019
(73) Titulaire: Institut des Sciences et Industries du Vivant et de l'Environnement - AgroParisTech, 75231 Paris Cedex 05 (FR)
(72) Inventeur: ALLAIS, Florent, 51400 BOUY (FR); BONNEAU, Guillaume, 51100 REIMS (FR); PERU, Aurélien, 51100 REIMS (FR); FLOURAT, Amandine, 51100 REIMS (FR)
(74) Mandataire: IP Trust
(86) Numéro de dépôt international: PCT/FR2017/051848
(87) Numéro de publication internationale: WO 2018/007764

(56) Documents cités:
- WO-A1-2014/147271
- WO-A1-2015/165957
- WO-A2-02/36090
- XINGHUA MA ET AL: "The Conversion of Levoglucosenone into Isolevoglucosenone", AUSTRALIAN JOURNAL OF CHEMISTRY: AN INTERNATIONAL JOURNAL FOR CHEMICAL SCIENCE, vol. 68, no. 4, 1 janvier 2015 (2015-01-01), page 593, XP055314009, AU ISSN: 0004-9425, DOI: 10.1071/CH14574
- KOSEKI KOSHI ET AL: "A method for easy preparation of optically pure (S)-5-hydroxy-2-penten-4-olide and (S)-5-hydroxypentan-4-olide", HETEROCYCLES COMMUNICATION | SPECIAL ISSUE, JAPAN INSTITUTE OF HETEROCYCLIC CHEMISTRY, JP, vol. 31, no. 3, 1 janvier 1990 (1990-01-01), pages 423-426, XP008176618, ISSN: 0385-5414, DOI: 10.3987/COM-89-5300

## Description

La présente invention se rapporte au domaine de la transformation de la lévoglucosénone (LGO) en intermédiaires chimiques utilisés dans l'industrie agro-alimentaire et pharmaceutique. Ces intermédiaires chimiques obtenus à partir de la lévoglucosénone sont 4-hydroxyméthylbutyrolactone (2H-HBO) et 4-hydroxyméthylbutenolide (HBO).

Afin de résoudre le problème de dépendance vis-à-vis des énergies fossiles, il a été développé dans les dernières décennies des procédés de production de composés chimiques d'intérêt à partir de biomasse. En particulier, la biomasse lignocellulosique est devenue l'une des sources vertes de composés carbonés. La lévoglucosénone, de formule (Ia) : 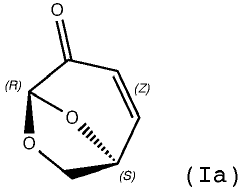 est l'un des produits les plus intéressants pouvant ainsi être obtenus à partir de biomasse, notamment par une technologie de flash pyrolyse de cellulose.

La lévoglucosénone est couramment employée en tant que produit de départ pour la synthèse de divers composés chimiques d'intérêt, en particulier de 4-hydroxyméthylbuténolide (HBO), de formule (IIa), et de 4-hydroxyméthylbutyrolactone (2H-HBO), de formule (IIb) :

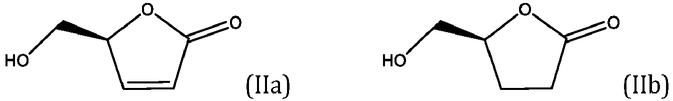

Ces composés présentent un intérêt particulier car ils constituent des intermédiaires chimiques asymétriques (chiraux) à forte valeur ajoutée qui sont fréquemment mis en œuvre dans l'industrie agroalimentaire, pour la production de fragrances et d'arômes, ou encore dans l'industrie pharmaceutique, pour la production de principes actifs de médicaments, tirant profit de leur noyau lactonique et de leur centre chiral.

La transformation de lévoglucosénone en 4-hydroxyméthylbuténolide s'effectue de manière classique en deux étapes successives, la première de ces étapes consistant en une réaction d'oxydation du type Baeyer-Villiger, pour former un intermédiaire formiate, suivie par une étape d'hydrolyse acide pour former le 4-hydroxyméthylbuténolide. Pour obtenir la 4-hydroxyméthylbutyrolactone, la lévoglucosénone est au préalable soumise à une étape d'hydrogénation catalytique, de sorte à former de la dihydrolévoglucosénone qui est ensuite soumise aux étapes de transformation décrites ci-avant en référence à la lévoglucosénone.

### Etat de la technique

Il est connu de l'état de la technique des procédés de transformation de la lévoglucosénone en 4-hydroxyméthylbutyrolactone et 4-hydroxyméthylbutenolide.

La transformation de lévoglucosénone en 4-hydroxyméthylbuténolide s'effectue de manière classique en deux étapes successives, la première de ces étapes consistant en une réaction d'oxydation du type Baeyer-Villiger, pour former un intermédiaire formiate, suivie par une étape d'hydrolyse acide pour former le 4-hydroxyméthylbuténolide. Pour obtenir la 4-hydroxyméthylbutyrolactone, la lévoglucosénone est au préalable soumise à une étape d'hydrogénation catalytique, de sorte à former de la dihydrolévoglucosénone qui est ensuite soumise aux étapes de transformation décrites ci-avant en référence à la lévoglucosénone.

Il a été proposé par l'art antérieur plusieurs procédés pour réaliser la réaction d'oxydation de la lévoglucosénone ou de la dihydrolévoglucosénone.

A titre d'exemples, on peut citer le document US 4,994,585, qui décrit l'oxydation de lévoglucosénone par un peracide, tel que l'acide m-chloroperbenzoïque ou l'acide peracétique, dans un solvant organique ; ou le document US 5,112,994, qui décrit un procédé de synthèse de 4-hydroxyméthylbutyrolactone à partir de dihydrolévoglucosénone, selon lequel la réaction d'oxydation est également réalisée par un peracide dans un solvant organique. Afin d'obtenir un rendement élevé, ces réactions doivent toutefois être conduites pendant une longue durée, de un à deux jours.

Il a autrement été proposé dans la publication de Paris et al., dans Green Chemistry, 2013, 15, 2101-2109, de réaliser la réaction d'oxydation de la lévoglucosénone par des catalyseurs métalliques tels que les zéolites d'aluminium ou d'étain, en présence d'un agent oxydant. De tels procédés peuvent cependant également être longs à mettre en œuvre, pour certains des catalyseurs proposés, pour obtenir un taux de conversion de la lévoglucosénone qui s'avère satisfaisant. Les zéolites d'aluminium permettent d'obtenir des taux de conversion élevés en quatre heures. Cependant, ce temps ne prend pas en compte le temps nécessaire à la préparation du catalyseur. Ce dernier s'avère en outre potentiellement toxique.

Il a par ailleurs été proposé par l'art antérieur, dans un objectif de meilleur respect de l'environnement par mise en œuvre de substances moins toxiques, de réaliser l'oxydation de cétones cycliques par réaction de type Baeyer-Villiger au moyen, en tant que catalyseur, d'une lipase immobilisée sur un support particulier en silice poreuse greffée. Un tel procédé est notamment décrit dans la publication de Drozdz et al., dans Applied Catalysis A: General, 2013, 467, 163-170. Comme décrit dans ce document, ce procédé s'avère satisfaisant, en termes de taux de conversion de la cétone cyclique et de temps de réaction, pour les cétones à forte tension de cycle, telles que la cyclobutanone ou la cyclopentanone, mais uniquement pour les cétones cycliques de ce type. Au contraire, pour les cétones cycliques à faible tension de cycle, telles que la cyclohexanone ou la cycloheptanone, une telle réaction d'oxydation s'avère peu performante, car permettant d'obtenir de faibles taux de conversion, et/ou en des temps très longs. Une telle différence de réactivité en fonction de la tension de cycle, liée notamment au nombre de chaînons du cycle, est bien connue de l'homme du métier, dont les connaissances générales en la matière sont notamment illustrées par le document de Wiberg, dans Angew. Chem. Int. Ed. Engl., 1986, 25, 312-322.

Le document WO2015/165957 décrit un procédé de transformation de la lévoglucosénone en 4-hydroxyméthylbutyrolactone et 4-hydroxyméthylbutenolide, comprenant une étape d'oxydation de la lévoglucosénone, ou de dihydrolévoglucosénone obtenue par hydrogénation de lévoglucosénone, par mise en contact d'une solution de lévoglucosénone ou de dihydrolévoglucosénone dans un solvant, avec une lipase en présence d'un agent oxydant et d'un composé donneur d'acyle. Cette étape d'oxydation est suivie d'une étape d'hydrolyse du mélange réactionnel obtenu, ainsi que, le cas échéant, d'une étape d'hydrogénation du composé obtenu à l'issue de cette étape d'hydrolyse.

### Inconvénients de l'art antérieur

Les solutions de l'art antérieur impliquent toutes une synthèse en deux étapes pouvant aller jusqu'à deux jours. Les solutions de l'art antérieur mettent toujours en œuvre un solvant et un catalyseur. L'Homme du métier est donc systématiquement conduit à réaliser la transformation de la lévoglucosénone en 4-hydroxyméthylbutyrolactone et 4-hydroxyméthylbutenolide en deux étapes en présence de solvant et de catalyseur. Ce catalyseur dans l'état de la technique est soit chimique, par exemple des zéolites, soit biologique, par exemple une enzyme de type lipase. Or ces solvants et catalyseurs, même après traitement de purification, demeurent à l'état de traces dans le produit fini. Le produit obtenu ne présente donc pas un niveau de pureté idéal. Ces traces entraînent une toxicité, particulièrement pour les catalyseurs métalliques de type étain, pouvant causer in fine des problèmes de santé chez le consommateur. De même, ces traces peuvent altérer la performance des synthèses de molécules dont le HBO et le 2H-HBO sont des intermédiaires de synthèse.

### Solution apportée par l'invention

Il a été constaté par les présents inventeurs, de manière tout à fait inattendue, que la transformation de la lévoglucosénone en 4-hydroxyméthylbutyrolactone et 4-hydroxyméthylbutenolide pouvait être réalisée en une seule étape en l'absence de solvant organique et de catalyseur, tout en ayant de bon taux de rendement. La solution de la présente invention est donc non toxique, plus respectueuse de l'environnement, simple et présente de bons rendements. Les rendements obtenus avec le procédé selon l'invention sont identiques voire meilleurs que ceux obtenus avec les procédés de l'art antérieur utilisant des catalyseurs chimiques ou biologiques. De plus, le procédé selon l'invention peut être réalisé en flux continu. L'absence de solvant organique et de catalyseur dans le procédé selon l'invention permet d'obtenir des produits d'un niveau de pureté supérieur à celui obtenu avec les procédés de l'art antérieur. Ceci est particulièrement intéressant étant donné que ces produits sont des intermédiaires utilisés en agroalimentaire, notamment à destination de l'alimentation humaine. Ceci limite l'impact sur l'environnement et sur la santé. Ceci limite également l'impact des impuretés sur les synthèses ultérieures comparé aux procédés de l'art antérieur. De plus le procédé selon l'invention est particulièrement économique.

### DESCRIPTION DETAILLEE DE L'INVENTION

La présente invention se propose dans son acceptation générale de remédier aux inconvénients de l'art antérieur par un procédé de transformation de la lévoglucosénone en un composé de formule générale (II) : 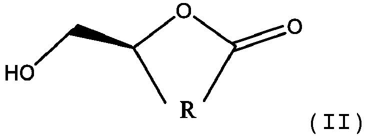 dans laquelle R représente -CH=CH- ou -CH₂-CH₂-, comprenant les étapes successives suivantes :
a) le cas échéant, pour obtenir un composé de formule générale (II) dans laquelle R représente -CH₂-CH₂-, hydrogénation de la lévoglucosénone pour former de la dihydrolévoglucosénone,
b) oxydation de la lévoglucosénone ou de la dihydrolévoglucosénone obtenue à l'étape a),
c) hydrolyse du mélange réactionnel obtenu à l'étape b),
d) le cas échéant, pour obtenir un composé de formule générale (II) dans laquelle R représente -CH₂-CH₂-, si une étape a) n'a pas été réalisée, hydrogénation du composé obtenu à l'étape c),
caractérisé en ce que l'oxydation de l'étape b) et l'hydrolyse de l'étape c) sont réalisées en une seule étape en présence d'une solution aqueuse d'H₂0₂, sans impliquer de solvant organique, ni de catalyseur.

Le procédé selon l'invention permet en une seule et unique réaction, l'oxydation de Bayer-Villiger et l'hydrolyse du formate intermédiaire. Contrairement aux solutions de l'art antérieur, l'hydrolyse ne nécessite pas la mise en œuvre d'une seconde étape réactionnelle utilisant des acides et des solvants organiques. Contrairement aux solutions de l'art antérieur utilisant pour faire la réaction de Bayer-Villiger sur la lévoglucosénone ou la dihydrolévoglucosénone de l'H₂0₂, un catalyseur (chimique tel que des zéolites ou biologique tel qu'une lipase) et un solvant organique (tel que le 1,4-dioxane ou l'acétate d'éthyle), le procédé selon l'invention n'utilise que de l'H₂0₂. L'H₂0₂ est sous forme de solution aqueuse d'H₂0₂, d'H₂O₂-urée ou de 2Na₂CO₃.3H₂O₂. De façon totalement inattendue, dans le procédé selon l'invention, l'hydrolyse se fait en même temps que l'oxydation.

Le procédé selon l'invention utilise une solution aqueuse d'H₂0₂, l'eau jouant le rôle de solvant, parfaitement non toxique.

La réaction selon l'invention exclut donc la présence de tout type de solvant organique et inorganique, à l'exception de l'eau.

Par « solvant organique » au sens de l'invention, on entend des solvants contenant des atomes de carbone. Les solvants organiques sont classés en trois familles :
1) les solvants hydrocarbonés qui comprennent les solvants aliphatiques tels que les alcanes et les alcènes, les solvants aromatiques tel que le benzène, le toluène et le xylène ;
2) les solvants oxygénés tels que les alcools (ex : éthanol, méthanol), les cétones (ex : acétone, MiBK), les acides (ex : acide acétique), les esters (ex : acétate d'éthyle), les éthers (ex : éther, éthers de glycol et autres solvants (ex: DMF, DMSO et HMPT) ;
3) les solvants halogénés tels que les hydrocarbures halogénés c'est-à-dire fluorés, chlorés, bromés ou iodés (ex: perchloroéthylène, trichloréthylène, dichlorométha ne, chloroforme, tétrachlorométhane).

Par « solvants inorganiques » au sens de l'invention, on entend des solvants ne contenant pas d'atome de carbone. Outre l'eau, les solutions aqueuses contenant des additifs (notamment des tensioactifs), l'acide sulfurique concentré, l'ammoniaque sont des solvants inorganiques classiques.

La réaction d'hydrogénation peut être effectuée par toute méthode connue de l'homme du métier, notamment par hydrogénation catalytique, par exemple en présence de palladium sur charbon et d'hydrogène.

Avantageusement, le temps de réaction en présence d'une solution aqueuse d'H₂0₂ est compris entre 2 et 53 heures.

Dans un mode de réalisation, la température de la réaction est de -78 °C à 50°C, de préférence de -78 °C à 40°C, voire de -78 °C à 20 °C, pendant l'ajout de la solution aqueuse d'H₂O₂ sur une durée de 5 minutes à 8 heures de manière à contrôler l'exothermicité de l'addition, puis la température de la réaction est comprise entre 2 °C et 150 °C pendant une durée de d'au moins 4 heures, notamment de 4 heures à 48h, de préférence de 8 heures à 48 heures une fois l'ajout de la solution d'H₂O₂ terminée.

Dans un mode préféré de réalisation, la température de la réaction est 0 °C pendant l'ajout de la solution aqueuse d'H₂O₂ sur une durée de 3,5 heures, voire de 4 heures, puis la température de la réaction est de 50 °C pendant une durée de 20 heures, de préférence de 8 heures, une fois l'ajout de la solution d'H₂O₂ terminée.

La solution aqueuse d'H₂0₂ est d'une concentration comprise entre 20 et 60%.

De préférence, la solution aqueuse d'H₂0₂ est d'une concentration de 30% ou 50%.

Le nombre d'équivalents en H₂0₂ est compris en 0,8 et 10 par rapport au nombre de moles de lévoglucosénone de départ.

Dans un mode de réalisation, le nombre d'équivalents en H₂0₂ est de 1,2 par rapport au nombre de moles de lévoglucosénone de départ. Dans un mode de réalisation préféré, ce nombre d'équivalents est de 1.

Plus la quantité d'H₂0₂ est importante, plus la réaction est rapide.

La quantité de 1,2 équivalent est particulièrement avantageuse. Dans un mode de réalisation encore plus avantageux, cette quantité en équivalent est de 1. Ces quantités permettent d'obtenir un bon rendement avec un bon taux de conversion/rendement tout en minimisant la quantité de réactif. Cette quantité est plus économique. Cette faible quantité de H₂0₂ évite également une étape de neutralisation d'H₂0₂ en excès. Cette quantité de 1,2 , voire de 1 équivalent, est donc une bonne optimisation entre taux de conversion/rendement, temps de réaction, mise en oeuvre et coût.

De manière avantageuse, l'excès d'oxydant présent dans le milieu réactionnel peut être neutralisé par chauffage à haute température ; cette « haute température » est comprise entre 70 °C et 150 °C. De préférence, la température appliquée est comprise entre 80 °C et 100 °C, de manière encore plus préférée de 90 °C.

Avantageusement, le procédé ne nécessite pas de solvants organiques (autre que l'eau) ni de catalyseurs (qu'ils soient métalliques ou biologiques).

Ainsi, le procédé selon l'invention permet de synthétiser des préparations de HBO et de 2H-HBO ne contenant pas de trace de métaux provenant de catalyseur métallique, ni de trace de protéines provenant de catalyseur biologique, ni de trace de solvant organique. Cette préparation présente une pureté jamais obtenue précédemment par les procédés décrits dans l'art antérieur.

La HBO et le 2H-HBO ainsi disponibles dans des préparations dépourvues de contaminants toxiques, sont particulièrement appropriées pour la préparation de molécules dérivées qui seront utilisées en agroalimentaire, en cosmétique ou en thérapeutique.

La préparation de HBO et de 2H-HBO peut ainsi être utilisée dans des applications à visée agroalimentaire, cosmétique ou thérapeutique.

La présente invention sera mieux comprise à la lumière d'exemples non limitatifs de réalisation.

### BREVES DESCRIPTION DES FIGURES

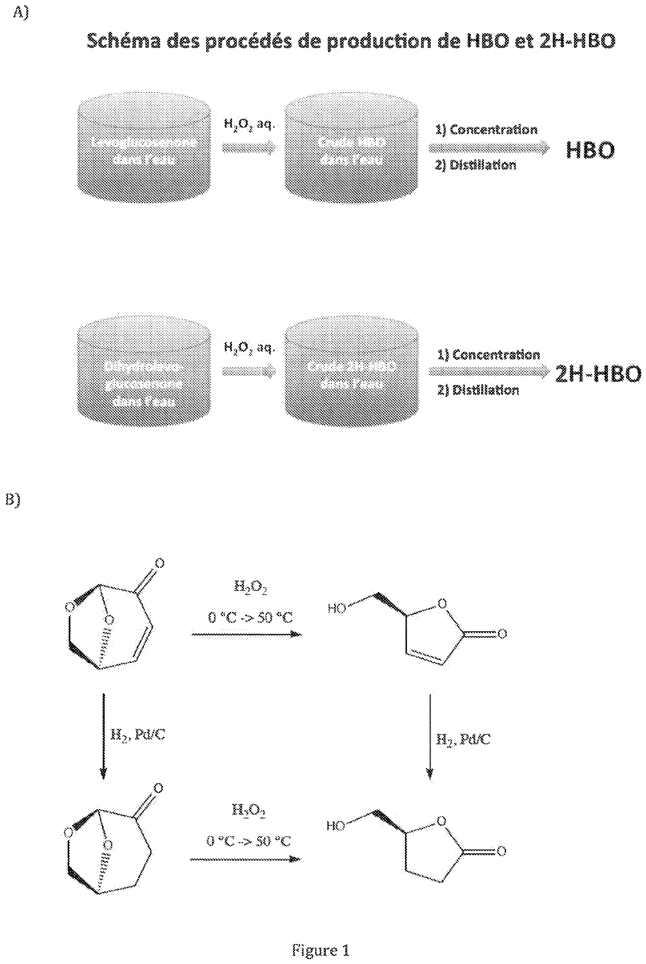

La figure 1 représente le schéma de la synthèse selon l'invention. (A) Représentation simplifiée, (B) Représentation de la réaction au niveau de la structure des molécules.
La figure 2 représente le pourcentage de HBO formé au cours du temps en fonction de la quantité de H₂0₂ dans le mélange réactionnel.
La figure 3 représente le suivi par HPLC de la formation de ε-caprolactone selon l'exemple 5.

### EXEMPLES

### Exemple 1 : synthèse de 4-hydroxyméthylbuténolide (HBO)

### A- Réaction impliquant un nombre équivalent en H₂O₂ de 1,2 par rapport au nombre de mole de LGO de départ

Une solution aqueuse de peroxyde d'hydrogène H₂0₂ à 30% (9,78 M, 25 mL, 0,245 mol 1.2 équivalent par rapport à la lévoglucosénone) est ajoutée goutte par goutte à l'aide d'une pompe seringue (8 mL/h) sous azote pendant 3h30 à la lévoglucosénone (25,7 g) sous agitation dans un bac contenant un mélange eau-glace. Puis le mélange réactionnel est chauffé à 50 °C pendant 20h sous agitation.

L'excès d'oxydant est neutralisé à basse température (à l'aide d'un bac contenant un mélange eau-glace) en ajoutant de petites quantités de sulfite de sodium (3 g, 23,8 mmol) sous agitation. Ceci est contrôlé par des bandelettes de test au peroxyde.

Le sulfite de sodium peut être remplacé par du bisulfite de sodium, du metabisulfite de sodium ou une catalase.

De manière avantageuse, l'excès d'oxydant peut alternativement être neutralisé par chauffage à haute température, par exemple à environ 90 °C. En procédant ainsi on évite l'utilisation de « neutraliseurs » qu'il faudra éliminer par la suite.
- extraction - purification :
   La purification peut être exécutée comme suit : 150 mL de toluène est ajouté au mélange réactionnel et concentré jusqu'à séchage. Le résidu est repris dans de l'acétone (25 mL) et filtré sur un tampon de célite, rincé avec 10 mL d'acétone. Le filtrat (injection directe de liquide) est purifié par chromatographie sur gel de silice (220 g, 30 micron) élué avec 50 à 100% d'acétate d'éthyle dans du cyclohexane pour donner 16,8 g de produit pur HBO (72%) sous forme d'huile incolore qui cristallise.

### B- Réaction impliquant un nombre équivalent en H₂O₂ de 1 par rapport au nombre de mole de LGO de départ:

Une solution aqueuse de peroxyde d'hydrogène H₂0₂ à 30% (9,78 M, 0,81 L, 7,92 mol 1 équivalent par rapport à la lévoglucosénone) est ajoutée goutte par goutte à l'aide d'une pompe seringue sur une période de 4 heures à une solution de lévoglucosénone (1 kg, 7,93 mol) dans 1 L d'eau sous agitation dans un bac contenant un mélange eau-glace. Puis le mélange réactionnel est chauffé à 50 °C pendant 6h sous agitation. L'absence de peroxyde résiduel est contrôlée par des bandelettes de test au peroxyde.
- extraction - purification :
   La purification peut être exécutée selon la méthode décrite au point A. Dans cet exemple, le mélange réactionnel a été concentré sous vide puis purifié par distillation pour donner 647 g de produit pur HBO (71%) sous forme d'huile incolore qui cristallise.

### Exemple 2 : synthèse de 4-hydroxyméthylbutyrolactone (2H-HBO)

### - voie 1 par hydrogénation catalytique de la HBO obtenue à l'exemple 1 :

Le 4-hydroxyméthylbuténolide (HBO) (IIa) obtenu à l'Exemple 1 est soumis à hydrogénation catalytique, de la manière suivante.

Du Pd/C (10 % p/p, 140 mg) est ajouté à une solution de 4-hydroxyméthylbuténolide (1,4 g, 12,3 mmol) dans de l'acétate d'éthyle (15 mL) à température ambiante. La suspension sous agitation est dégazée 3 fois sous vide/azote. La suspension est ensuite hydrogénée par une atmosphère d'hydrogène à température ambiante pendant 4 heures. Le mélange brut est filtré sur célite et le filtrat est concentré à sec. Le produit brut est purifié par distillation ou chromatographie sur gel de silice (élution avec un gradient allant de 75 à 100% d'acétate d'éthyle dans le cyclohexane), pour obtenir de la 4-hydroxyméthylbutyrolactone (2H-HBO) de formule (IIb) pure (1,19 g, 82%).

¹H NMR (CDCl₃, 300 MHz): δ_{H} 2.20 (m, 2H), 2.61 (m, 3H), 3.66 (dd, 1H, *J* = 12.6 and 4.5 Hz), 3.92 (dd, 1H, *J* = 12.6 and 2.7 Hz), 4.64 (m, 1H)

¹³C NMR (CDCl₃, 75 MHz): δ_{c} 23.1 (t), 28.7 (t), 64.1 (t), 80.8 (d), 177.7 (s)

### - Voie 2 par hydrogénation de la lévoglucosénone puis synthèse :

o Hydrogénation catalytique de la lévoglucosénone :
   Du Pd/C (10 % p/p, 500 mg) est ajouté à une solution de (-)-lévoglucosénone LGO (5 g, 39,7 mmol) dans de l'acétate d'éthyle (50 mL) à température ambiante. La suspension sous agitation est dégazée 3 fois sous vide/azote. La suspension est ensuite
   hydrogénée par une atmosphère d'hydrogène à température ambiante jusqu'à consommation totale du produit de départ, soit environ 4 h. Le mélange brut est filtré sur célite et le filtrat est concentré à sec. Le produit brut est purifié par distillation ou
   chromatographie sur gel de silice (élution avec 10 à 60% d'acétate d'éthyle dans le cyclohexane), pour obtenir de la dihydrolévoglucosénone de formule (Ib) (2H-LGO) pure (huile incolore, 4,4 g, 87%).
   ¹H NMR (CDCl₃, 300 MHz): δ_{H} 2.02 (m, 1H), 2.34 (m, 2H), 2.62 (m, 1H), 4.00 (m, 2H), 4.70 (m, 1H), 5.10 (s, 1).
   ¹³C NMR (CDCl₃, 75 MHz): δ_{c} 29.9 (t), 31.1 (t), 67.5 (t), 73.1 (d), 101.5 (d), 200.3 (s)
o Réaction de Baeyer-Villiger :
   Option 1 : Une solution aqueuse de peroxyde d'hydrogène H₂0₂ à 30% (9,78 M, 25 mL, 0,245 mol 1,2 équivalent par rapport à la dihydrolévoglucosénone) est ajoutée goutte par goutte à l'aide d'une pompe seringue (8 mL/h) sous azote pendant 3h30 à la dihydrolévoglucosénone (26,1 g) sous agitation dans un bac contenant un mélange eau-glace. Puis le mélange réactionnel est chauffé à 50 °C pendant 20h sous agitation. L'excès d'oxydant est neutralisé à basse température (à l'aide d'un bac contenant un mélange eau-glace) en ajoutant de petites quantités de sulfite de sodium (3 g, 23,8 mmol) sous agitation. Ceci est contrôlé par des bandelettes de test au peroxyde.

Extraction - purification :
150 mL de toluène sont ajoutés au mélange réactionnel et ce dernier est concentré jusqu'à séchage. Le résidu est repris dans de l'acétone (25 mL) et filtré sur célite, et rincé avec 10 mL d'acétone. Le filtrat (injection directe de liquide) est purifié par chromatographie sur gel de silice (220 g, 30 micron) élué avec 50 à 100% d'acétate d'éthyle dans du cyclohexane pour donner 16,8 g de produit pur 2H-HBO (72%) sous forme d'huile incolore qui cristallise.

L'étape d'extraction-purification de la 2H-HBO peut être réalisée en concentrant le mélange réactionnel sous vide puis en le purifiant par distillation, comme décrit précédemment pour la HBO.

Option 2 : Une solution aqueuse de peroxyde d'hydrogène H₂0₂ à 30% (9,78 M, 0,81 L, 7,92 mol 1 équivalent par rapport à la dihydrolévoglucosénone) est ajoutée goutte par goutte à l'aide d'une pompe seringue sur une période de 4 heures sous azote à une solution de dihydrolévoglucosénone (1,02 kg, 7,93 mol) dans 1 L d'eau sous agitation dans un bac contenant un mélange eau-glace. Puis le mélange réactionnel est chauffé à 50 °C pendant 6h sous agitation. L'absence de peroxyde résiduel est contrôlé par des bandelettes de test au peroxyde.

Extraction - purification :
L'étape d'extraction-purification de la 2H-HBO peut être réalisée en concentrant le mélange réactionnel sous vide puis en le purifiant par distillation, comme décrit précédemment pour la HBO.

L'une comme l'autre des voies de synthèse 1 et 2 ci-dessus permettent d'obtenir, de manière régiosélective et avec des rendements élevés, la 4-hydroxyméthylbutyrolactone (IIb, 2H-HBO), dont la structure est confirmée par RMN du proton et du carbone.

### Exemple 3 : suivi de la transformation de LGO en HBO

- suivi cinétique de la réaction par HPLC :
   Protocole d'HPLC :
   Un échantillon de 2,5 *µ*L de milieu réactionnel est dilué dans 1,5 mL d'acétonitrile. L'évolution de la réaction est suivie par HPLC et la formation d'HBO est suivie à 220 nm.

Les analyses sont réalisées sur une colonne C18 Thermo Scientific^{®} Syncronis^{®} aQ (250 x 4,6 mm, 5 *µ*m) dans les conditions suivantes : volume d'injection 10 *µ*L ; température du four 30 °C ; flux à 0,8 mL.min⁻¹ ; méthode d'élution : isocratique 85/15 eau/acétonitrile de 0 à 5 min, de 5 à 10 min gradient de 85/15 à 90/10 eau/acétonitrile, de 10 à 15 min isocratique 90/10 eau/acétonitrile, de 15 à 20 min gradient de 90/10 à 85/15 eau/acétonitrile ; enregistrement du spectre à 220 nm.

L'excès d'eau oxygénée en fin de réaction a été quenchée par une pointe de spatule de sodium sulfite (Na₂SO₃ + H₂O₂ - > Na₂SO₄ + H₂O) à température ambiante. La réaction a été agitée pendant 5 min.

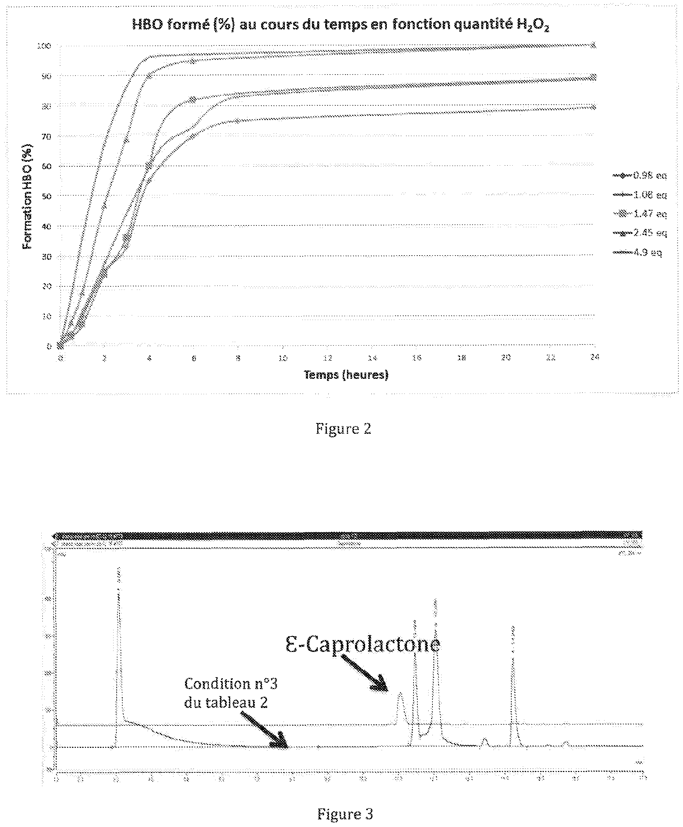

Des échantillons de 2,5 *µ*L sont prélevés dans le milieu réactionnel à différents intervalles de temps et analysés par HPLC selon le protocole décrit ci-avant. Différentes quantités d'H202 ont été comparées (figure 2). Les quantités d'H₂0₂ testées selon le procédé de synthèse de l'invention sont 0,98, 1,98, 1,47, 2,45 et 4,9 équivalent H₂0₂ par rapport à la quantité initiale du produit de départ. Plus la quantité de H₂0₂ est élevée plus la quantité de HBO formée est importante (figure 2).

### Exemple 4 : étude comparative du procédé selon l'invention et d'un procédé de l'art antérieur

La HBO a été synthétisée :
- selon le procédé de l'invention comme décrit ci-avant
- ainsi que par deux procédés de l'état de la technique :
   ∘ par la voie lipase telle que décrite dans le document A. L. Flourat, A. A. M. Peru, A. R. S. Teixeira, F. Brunissen, F. Allais, Green Chem., 2015, 17, 404-412 et dans la demande de brevet WO2015/165957
   ∘ par la voie zéolite telle que décrite dans le document C. Paris, M. Molier, A. Corma, Green Chem., 2013, 15, 2101-2109.
- Synthèse de HBO par la voie lipase :
   Oxydation :
   Dans un réacteur, une solution aqueuse de peroxyde d'hydrogène H₂O₂ à 30 % (2,57 mmol, 0,26 mL, 1,2 éq. par rapport à la LGO) est ajoutée en une unique portion à une suspension de LGO (270 mg, 2,14 mmol) et de lipase CaL-B (Novozym^{®} 435, 75 mg, 315 U/mmol LGO) dans l'acétate d'éthyle (3 mL) sous agitation à température ambiante, dans un incubateur à agitation planaire.
   Pour cet exemple comme pour tous les exemples décrits ci-après, 1 g de Novozym^{®} 435 correspond à 9000 unités de lipase CaL-B (activité mesurée après séjour de l'enzyme dans l'acétate d'éthyle). Le mélange réactionnel est agité à 40 °C pendant 4 h puis évaporé à sec.

Hydrolyse acide :
De l'acide chlorhydrique concentré (5 mmol, 0,4 mL) est ajouté à une solution de ce mélange brut dans le méthanol (5 mL), à température ambiante. Le mélange réactionnel est chauffé sous agitation pendant 8 à 16 heures, de sorte à convertir le formiate (IIIa) en l'alcool (IIa) correspondant. Le mélange réactionnel est évaporé à sec avec un gel de silice. Le produit brut est purifié par chromatographie sur gel de silice (élution avec 75 à 100% d'acétate d'éthyle dans du cyclohexane) pour obtenir du 4-hydroxyméthylbuténolide (IIa) pur (175 mg, 72%).

¹H RMN (CDCl₃, 300 MHz) : 7,53 (dd, *J* = 1,5 et 5,7 Hz, 1 H), 6,2 (dd, *J* = 1,5 et 5,7 Hz, 1 H), 5,17 (m, 1 H), 4,0 (d, *J* = 3,6 et 12,0 Hz, 1 H), 3,80 (dd, *J* = 3,6 et 12,0 Hz, 1 H), 3,25 (s, 1 H)

¹³C RMN (CDCl₃, 75 MHz): 173,5 (s), 154,0 (d), 122,8 (d), 84,3 (d), 62,2 (t)
- Synthèse de HBO par la voie zéolite :
   Oxydation :
      Dans un réacteur, la lévoglucosénone (0,33 mmol, 42 mg) est dissoute dans 1 mL de 1,4-dioxane et une solution de peroxyde d'hydrogène (35% dans l'eau, 0,5 mmol) est ajoutée à la solution.
      Ce mélange est ensuite mis en contact avec les zéolites (lévoglucosénone/métal de la zéolite = 20) et la réaction est maintenue à 100 °C pendant 4 heures. La solution est ensuite filtrée
   Hydrolyse acide :
      La solution préalablement filtrée est mise en contact avec 140 mg de résine acide Amberlyst-15 à température ambiante pendant 6 heures.
- Comparaison des trois techniques :
   La comparaison des trois procédés de synthèse (tableau 1) montre que la synthèse par le procédé selon l'invention se réalise en une seule étape alors que les deux autres procédés se font en deux étapes.

Le rendement du procédé de l'invention est de 72%, ce qui est supérieur au rendement obtenu par voie lipase. Le rendement par voie zéolite est de 89% mais celui-ci n'est pas isolé contrairement au rendement du procédé conformément à l'invention. Ainsi, le rendement isolé du procédé selon l'invention est supérieur aux rendements des techniques de l'état de la technique.

Le procédé selon l'invention n'implique qu'un seul réactif, l'H₂0₂, alors que les voies lipase et zéolite nécessitent un grand nombre de réactifs. Le procédé selon l'invention est donc plus avantageux et plus économique par rapport aux procédés de l'état de la technique.

Le procédé selon l'invention ne met pas en œuvre de grandes quantités de solvant organique ni de catalyseur métallique contrairement aux procédés de l'état de la technique. Il est donc plus respectueux de l'environnement. Le produit obtenu a également un niveau de pureté plus important et ne contient de fait pas de traces de solvant organique ni de catalyseur métallique. Ceci est particulièrement intéressant étant donné que la HBO et la 2H-HBO sont des intermédiaires utilisés dans la synthèse d'arômes pour les industries agroalimentaire et pharmaceutiques. Ceci limite donc les potentiels effets secondaires, tels que les risques d'allergies ou de toxicité, pour le consommateur final.

**Tableau 1. Comparaison du procédé de synthèse selon l'invention et de deux procédés de l'état de la technique**

| | **H₂O₂ seule** | **Voie Lipase** | **Voie Zéolite** |
|---|---|---|---|
| **Rendement isolé** | 72 % | 67% | 89 % (non isolé) |
| **Temps de réaction** | 23 heures 30 | 1^{ère} étape : 2 heures 2^{ème} étape : 6-8 heures | 1^{ère} étape : 4 heures 2^{ème} étape : 6 heures |
| **Température** | 3 heures 30 : 0 °C 20 heures : 50 °C | 40 °C | 100 °C |
| **Nombres d'étapes** | 1 | 2 | 2 |
| **Quantité de réactifs** | 1.2 équivalent H₂O₂ (30%) | CAL-B (quantité catalytique) Tampon solide (20 mg/mL) 1.2 équivalent H₂O₂ (50%) Amberlyst 15-H (420 mg/mmol de LGO) | 1.5 équivalent H₂O₂ (35%) Catalyseur Sn-Beta Amberlyst 15-H |
| **Solvant** | Pas de solvant organique | Acétate d'éthyle (C = 0,5-1M) | 1,4-dioxane (C = 0,33 M) |

### Exemple 5 : le procédé selon l'invention ne fonctionne pas avec n'importe quelle cétone

Le procédé selon l'invention est spécifique de la lévoglucosénone. Il n'est pas possible d'utiliser n'importe quelle cétone en tant que substrat.

Le procédé selon l'invention a été testé sur une cétone classique, la cyclohexanone. La lévoglucosénone et la cyclohexanone sont des cétones cycliques dont le cycle est à 6 carbones.

Différentes quantités de solution aqueuse de peroxyde d'hydrogène H₂0₂ à 30% ont été testées, avec ou sans solvants et également avec un catalyseur enzymatique tel que décrit dans le document WO2015/165957.

Une solution aqueuse de peroxyde d'hydrogène H₂0₂ à 30% (1,2 équivalent par rapport à la cyclohexanone, 0,59 mL, 5,8 mmol ou 2,5 équivalent par rapport à la cyclohexanone, 1,2 mL, 12,1 mmol) est ajoutée en une fois à une solution de cyclohexanone (0,5 mL, 4,84 mmol) sous agitation et refroidie dans un bac contenant un mélange eau-glace avec (condition 2) ou sans (condition 1 correspondant aux conditions de la demande WO2015/165957) éthanol (2 mL). Le mélange est laissé au froid sur le bac de mélange eau-glace pendant une heure. Puis le mélange réactionnel est chauffé à 50 °C pendant 18h sous agitation.

Pour la condition 2, un aliquot (0,5 mL) est prélevé et maintenu sous agitation avec du sulfite de sodium au froid sur un bac de mélange eau-glace. Ce mélange est ensuite dilué avec de l'éthanol, filtré et concentré jusqu'à obtenir un produit sec. La consommation du substrat de départ est suivie par TLC (chromatographie sur couche mince) (élution 7/3 cyclohexane/acétate d'éthyle, puis révélation par une solution de vanilline).

La formation de ε-caprolactone est suivie par HPLC (figure 3), chromatographie en phase liquide à haute performance, (Thermofisher Ultimate 3000 équipé d'un détecteur DAD) en utilisant une colonne de chromatographie C18 (Syncronis aQ 250 x 4,6 mm, 5 *µ*m). 50*µ*L d'échantillons sont ajoutés à 0,5 mL d'acétonitrile. 30 *µ*L de ces mélanges sont injectés dans le dispositif d'HPLC à une température de 30 °C sous un flux de 0,8 mL.min⁻¹. La méthode d'élution (eau-acétonitrile) est la suivante : 0-5 min 90/10, 5-10 min de 90/10 à 0/100, 10-20 min 0/100, 20-27 min de 0/100 à 90/10. Détecteur UV à 254 and 280 nm.

**Tableau 2. Le procédé selon l'invention ne fonctionne pas avec d'autres cétones.**

| **Conditions** | **H₂O₂ 30% eq** | **solvant** | **Commentaire** |
|---|---|---|---|
| 1 (procédé selon l'invention) | 1.2 | - | Pas de réaction |
| 2 | 1.2 | EtOH (2,4M) | Pas de réaction |
| 3 | 2.5 | - | Disparition partielle du produit de départ Nouveau produit observé en TLC et analysé en ¹H NMR et HPLC: pas de présence de ε-caprolactone |
| 4 | 2.5 | EtOH (2,4 M) | Pas de réaction |
| 5 (WO2015/165957) ^{∗} | 1.1 | Acétate d'éthyle +N435 (lipase) | 40% du produit de départ consommé après 24 heures (GCMS) |

| | | | |
|---|---|---|---|
| *(Conditions de la demande de brevet WO2015/165957: le mélange réactionnel est composé de 25 mg de N435 (525 U/mmol), 0,5 mmol cyclohexanone and 0,6 mmol H₂O₂ et d'acétate d'éthyle à un volume final de 1 mL. La réaction est réalisée pendant 48 h à 40 °C sous agitation de 400 rpm. | | | |

Le procédé de transformation qui fonctionne très bien sur la LGO ne fonctionne pas du tout sur la cyclohexanone (figure 3). La figure 3 montre que la ε-caprolactone a normalement un temps de rétention de 11 min. Dans les conditions n°3 du tableau 2, il n'y a pas de formation de ε-caprolactone. Le procédé selon l'invention ne fonctionne donc pas sur n'importe quelle cétone et est spécifique à la lévoglucosénone.

## Revendications

1. - Procédé de transformation de la lévoglucosénone en un composé de formule générale (II) : 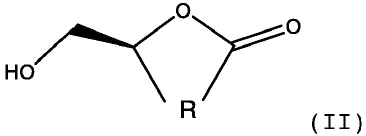 dans laquelle R représente -CH=CH- ou -CH₂-CH₂-, comprenant les étapes successives suivantes :
a) le cas échéant, pour obtenir un composé de formule générale (II) dans laquelle R représente -CH₂-CH₂-, hydrogénation de la lévoglucosénone pour former de la dihydrolévoglucosénone,
b) oxydation de la lévoglucosénone ou de la dihydrolévoglucosénone obtenue à l'étape a),
c) hydrolyse du mélange réactionnel obtenu à l'étape b),
d) le cas échéant, pour obtenir un composé de formule générale (II) dans laquelle R représente -CH₂-CH₂-, si une étape a) n'a pas été réalisée, hydrogénation du composé obtenu à l'étape c),
**caractérisé en ce que** l'oxydation de l'étape b) et l'hydrolyse de l'étape c) sont réalisées en une seule étape en présence d'une solution aqueuse d'H₂0₂, sans impliquer de solvant organique, ni de catalyseur.

2. - Procédé selon la revendication 1 **caractérisé en ce que** le temps de réaction en présence d'une solution aqueuse d'H₂0₂ est compris entre 2 et 53 heures.

3. - Procédé selon la revendication 1 ou 2 **caractérisé en ce que** la température de la réaction est de -78 °C à 50 °C pendant l'ajout de la solution aqueuse d'H₂O₂ sur une durée de 5 minutes à 8 heures, puis la température de la réaction est comprise entre 20 °C et 150 °C pendant une durée de 4 heures à 48 heures une fois l'ajout de la solution d'H₂O₂ terminée.

4. - Procédé selon l'une quelconque des revendications 1 à 3 **caractérisé en ce que** la température de la réaction est 0 °C pendant l'ajout de la solution aqueuse d'H₂O₂ sur une durée de 4 heures, puis la température de la réaction est maintenue à 0 °C pendant 8 heures une fois l'ajout de la solution d'H₂O₂ terminée puis de 50 °C pendant une durée de 8 heures.

5. - Procédé selon l'une quelconque des revendications 1 à 4 **caractérisé en ce que** la solution aqueuse d'H₂0₂ est d'une concentration comprise en 20 et 60%.

6. - Procédé selon l'une quelconque des revendications 1 à 5 **caractérisé en ce que** la solution aqueuse d'H₂0₂ est d'une concentration de 30% ou 50%.

7. - Procédé selon l'une quelconque des revendications 1 à 6 **caractérisé en ce que** le nombre d'équivalents en H₂0₂ est compris en 0,8 et 10 par rapport au nombre de mole de lévoglucosénone de départ.

8. - Procédé selon la revendication 7 **caractérisé en ce que** le nombre d'équivalents en H₂0₂ est de 1 par rapport au nombre de mole de lévoglucosénone de départ.

9. - Procédé selon l'une des revendications 1 à 8 comprenant en outre une étape de neutralisation de l'excès d'oxydant, cette étape étant réalisée par chauffage.

## Patentansprüche

1. Verfahren zum Umwandeln von Levoglucosenon in eine Verbindung der allgemeinen Formel (II): 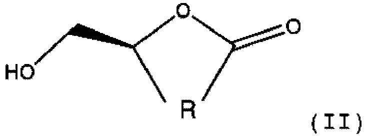 wobei R -CH=CH- oder -CH₂-CH₂- darstellt, umfassend die folgenden aufeinanderfolgenden Schritte:
a) gegebenenfalls, um eine Verbindung der allgemeinen Formel (II) zu erhalten, wobei R -CH₂-CH₂- darstellt, Hydrieren von Levoglucosenon, um Dihydrolevoglucosenon zu bilden,
b) Oxidieren des in Schritt a) erhaltenen Levoglucosenons oder des Dihydrolevoglucosenons,
c) Hydrolysieren des in Schritt b) erhaltenen Reaktionsgemisches,
d) gegebenenfalls, um eine Verbindung der allgemeinen Formel (II) zu erhalten, wobei R -CH₂-CH₂- darstellt, falls Schritt a) nicht durchgeführt wurde, Hydrieren der in Schritt c) erhaltenen Verbindung,
**dadurch gekennzeichnet, dass** das Oxidieren aus Schritt b) und das Hydrolysieren aus Schritt c) in einem einzigen Schritt in Gegenwart einer wässrigen H₂O₂-Lösung durchgeführt werden, ohne dass ein organisches Lösungsmittel oder ein Katalysator verwendet wird.

2. Verfahren nach Anspruch 1, **dadurch gekennzeichnet, dass** die Reaktionszeit in Gegenwart einer wässrigen H₂O₂-Lösung zwischen 2 und 53 Stunden beträgt.

3. Verfahren nach Anspruch 1 oder 2, **dadurch gekennzeichnet, dass** die Reaktionstemperatur während der Zugabe der wässrigen H₂O₂-Lösung über einen Zeitraum von 5 Minuten bis 8 Stunden -78 °C bis 50 °C beträgt, dann die Reaktionstemperatur während eines Zeitraums von 4 Stunden bis 48 Stunden zwischen 20 °C und 150 °C beträgt, sobald die Zugabe der H₂O₂-Lösung beendet ist.

4. Verfahren nach einem der Ansprüche 1 bis 3, **dadurch gekennzeichnet, dass** die Reaktionstemperatur während der Zugabe der wässrigen H₂O₂-Lösung über einen Zeitraum von 4 Stunden 0 °C beträgt, dann die Reaktionstemperatur während 8 Stunden bei 0 °C, sobald die Zugabe der H₂O₂-Lösung beendet ist, dann während eines Zeitraums von 8 Stunden bei 50 °C gehalten wird.

5. Verfahren nach einem der Ansprüche 1 bis 4, **dadurch gekennzeichnet, dass** die wässrige H₂O₂-Lösung eine Konzentration zwischen 20 und 60 % aufweist.

6. Verfahren nach einem der Ansprüche 1 bis 5, **dadurch gekennzeichnet, dass** die wässrige H₂O₂-Lösung eine Konzentration von 30 % oder 50 % aufweist.

7. Verfahren nach einem der Ansprüche 1 bis 6, **dadurch gekennzeichnet, dass** die Zahl der H₂O₂-Äquivalente, bezogen auf die Molzahl des Ausgangs-Levoglucosenons, zwischen 0,8 und 10 beträgt.

8. Verfahren nach Anspruch 7, **dadurch gekennzeichnet, dass** die Zahl der H₂O₂-Äquivalente, bezogen auf die Molzahl des Ausgangs-Levoglucosenons, 1 beträgt.

9. Verfahren nach einem der Ansprüche 1 bis 8, das ferner einen Schritt des Neutralisierens des überschüssigen Oxidationsmittels umfasst, wobei dieser Schritt durch Erhitzen durchgeführt wird.

## Claims

1. Method for converting levoglucosenone into a compound of general formula (II): 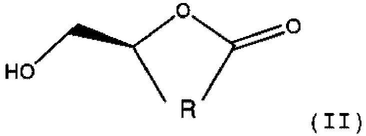 where R represents -CH=CH- or -CH₂-CH₂-, comprising the following successive steps:
a) where appropriate, in order to obtain a compound of general formula (II), where R represents -CH₂-CH₂-, hydrogenating levoglucosenone to form dihydrolevoglucosenone,
b) oxidizing the levoglucosenone or the dihydrolevoglucosenone obtained in step a),
c) hydrolyzing the reaction mixture obtained in step b),
d) where appropriate, in order to obtain a compound of general formula (II), where R represents -CH₂-CH₂-, if step a) has not been carried out, hydrogenating the compound obtained in step c),
**characterized in that** the oxidation in step b) and the hydrolysis in step c) are carried out in a single step in the presence of an aqueous solution of H₂O₂, without involving any organic solvent or catalyst.

2. Method according to claim 1, **characterized in that** the reaction time in the presence of an aqueous solution of H₂O₂ is between 2 and 53 hours.

3. Method according to either claim 1 or claim 2, **characterized in that** the reaction temperature is -78°C to 50°C when the aqueous solution of H₂O₂ is added over a period of 5 minutes to 8 hours, and then the reaction temperature is between 20°C and 150°C for a period of 4 hours to 48 hours once the solution of H₂O₂ has been added.

4. Method according to any of claims 1 to 3, **characterized in that** the reaction temperature is 0°C when the aqueous solution of H₂O₂ is added over a period of 4 hours, and then the reaction temperature is maintained at 0°C for 8 hours once the solution of H₂O₂ has been added and is then maintained at 50°C for a period of 8 hours.

5. Method according to any of claims 1 to 4, **characterized in that** the aqueous solution of H₂O₂ has a concentration of between 20 and 60%.

6. Method according to any of claims 1 to 5, **characterized in that** the aqueous solution of H₂O₂ has a concentration of 30% or 50%.

7. Method according to any of claims 1 to 6, **characterized in that** the number of H₂O₂ equivalents is between 0.8 and 10 relative to the number of moles of starting levoglucosenone.

8. Method according to claim 7, **characterized in that** the number of H₂O₂ equivalents is 1 relative to the number of moles of starting levoglucosenone.

9. Method according to any of claims 1 to 8, further comprising a step of neutralizing the excess oxidant, this step being carried out by heating.
